# EUROPEAN PATENT APPLICATION

(11) **EP 3 750 512 A1**
(43) Date of publication of application: **16.12.2020**
(21) Application number: 19000291.5
(22) Date of filing: 13.06.2019
(51) Int. Cl.: A61F 5/453, A61F 5/455

(54) **A MENSTRUAL FLOW COLLECTING DEVICE**

(71) Applicant: Odlen, Ingrid, 227 34 Lund (SE)
(72) Inventor: Odlen, Ingrid, 227 34 Lund (SE)
(74) Representative: Quintelier, Claude

(57) **Abstract**

A menstrual flow collecting device comprising a retaining body and a collecting member made of expandable impermeable material, said collecting member being applied to said body, said body comprises a first and a second end located at opposite sides of said body and an outer and an inner wall, said first end being an open end, said collecting member having a bottom part, said collecting member being, in use, in frictional contact with at least part of said outer and inner wall, said collecting member being applied in such a manner to said body as to envelope said body.

## Description

The present invention relates to a menstrual flow collecting device comprising a retaining body and a collecting member made of expandable impermeable material, said collecting member being applied to said body, said body comprises a first and a second end located at opposite sides of said body and an outer and an inner wall, said first end being an open end, said collecting member having a bottom part.

Such a menstrual flow collecting device is known from US-A 2010/0312204. The known device comprises a bag shaped body, having a pulling stem at its second end. Inside this body a collecting member is removably applied. The body comprises a pulling stem at the second end, a circular lip at the first end, a circular groove below the circular lip and circular rib below the circular groove. The collecting member has a resilient restraining ring located in the circular groove of the body when the collecting member is fitted to the body. The circular rib is adapted to prevent the restraining ring of the member from being pushed out of the circular groove when the collecting member is removed from the vagina.

A drawback of the know menstrual flow collecting device is that it gives a rather uncomfortable feeling to a female person who carries it. The presence of the circular lip and of the circular rib, which form protrusions on the body, exert a pressure on the vagina which creates this uncomfortable feeling. Furthermore, the fact that the pulling stem, which is part of the body, has to be used for removing the device imposes that the body comes in contact with the fingers of the person removing the device. This finger contact can lead to contamination of the body and thus of the women or girl who carries the device, in particular when the body is re-used.

It is an object of the present invention to provide a menstrual flow collecting device which is comfortable to carry and where the risk of contamination is substantially reduced.

To this purpose a menstrual flow collecting device according to the invention is characterised in that said collecting member being, in use, in frictional contact with at least part of said outer and inner wall, said collecting member being applied in such a manner to said body as to envelope said body. As the body is enveloped by the collecting member there is no need to touch the body when applied to or removed from the vagina, thereby limiting considerably the probability of contamination, in particular when the body is re-used. The fact also that the collecting member is retained by the friction exerted on the body and that the body is enveloped by the collecting member, there is no need to provide additional means for retaining the collecting member in the body, which provides a comfort in carrying the device.

A first preferred embodiment of a menstrual flow collecting device according to the invention is characterised in that said collecting member is removably applied to said body. This enables to thrown away the collecting member after use. So, even if the collecting member would have been contaminated upon removal, the risk of contaminating the women or girl remains very limited as the collecting member can be thrown away.

A second preferred embodiment of a menstrual flow collecting device according to the invention is characterised in that said second end is also an open end and said collecting member has an entrance opening and being applied in such a manner to said body as to having its bottom part crossing said entrance opening. As the bottom parts crosses the entrance part, the bottom part can be used for removing the device.

Preferably said retaining body is vessel shaped. The vessel shape will contribute to give a vessel shape to the member when applied in the body, which on his turn will offer a suitable temporarily storage for the collected menstrual flow.

A third preferred embodiment of a menstrual flow collecting device according to the invention is characterised in that said collecting member is disposable. In such a manner the hygienic properties are increased and the risk of contamination is considerably reduced.

A fourth preferred embodiment of a menstrual flow collecting device according to the invention is characterised in that said collecting member is formed by a condom. Condom are by nature hygienic and very well compatible for human use.

A fifth preferred embodiment of a menstrual flow collecting device according to the invention is characterised in that said condom is one having passed his expiration date. Although the expiration date has expired, the condom still remains useful as member for collecting the menstrual flow. Moreover, this option opens a possibility to still make use of the condom, thereby reducing the amount of waist produced.

A sixth preferred embodiment of a menstrual flow collecting device according to the invention is characterised in that characterised in that said body is made of elastic material enabling to fold or twist the body. This facilitates the insertion of the device into the vagina.

The invention will now be described in more details with reference to the annexed drawings showing preferred embodiments of a menstrual flow collecting device according to the invention. In the drawings:
Figure 1 shows an overall view of a first preferred embodiment of the body;
Figure 2 shows a cross-section through the body along the lines II-II';
Figure 3 shows a first preferred embodiment of the menstrual flow collecting device according to the invention; and
Figure 4 shows a second preferred embodiment of the menstrual flow collecting device according to the invention.

In the drawings a same reference sign has been allotted to a same or analogous element.

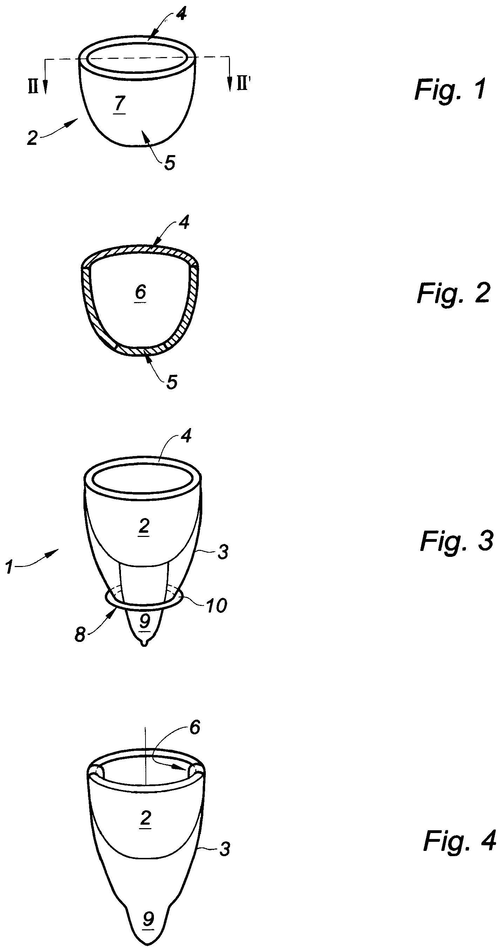

The menstrual flow collecting device 1 according to the invention and shown in figure 3 comprising a retaining body 2, which is vessel shaped, and a collecting member 3. The collecting member is made of expandable or elastic impermeable material. Preferably the collecting member is cup shaped and for example formed by a condom and more preferably by a condom having passed his expiration date, as will be described in more details hereunder.

Figures 1 and 2 show a view of the first embodiment of the retaining body, which in this embodiment is a vessel shaped body 2. The body comprises a first 4 and a second 5 end located at opposite sides of the body. The first and second ends being open ends. The body also comprises an outer 7 and an inner 6 walls. The body is preferably made of elastic material enabling to fold or twist the body. This enable to easily fold or twist the body which facilitates the application of the device into the vagina. The material of which the body is made is preferably silicone or rubber, which are materials having very good elastic properties, are very well compatible with the human body, easy to clean and enable moreover a cheap and reliable manufacture of the body.

In the embodiment illustrated in the figures 1 to 3, the body is conically shaped. The conical shape has the advantage to fit well with the shape of the vagina, thereby enabling an easy application and a comfort while carrying it. Alternatively, the body could also be funnel shaped, tube, or ring shaped.

As already mentioned, the collecting member 3 is preferably formed by a condom. The condom is tool which can easily be obtained in a large part of the world and is made of elastic and impermeable material. The use of a condom as a collecting member for collecting a menstrual flow of a female person has the advantage of using a well-known tool, largely available on the market on a price which is affordable for a very large number of people. As moreover the condom can be disposed after a single use as menstrual flow collecting member, it offers a hygienic solution thereby substantially reducing the risk of contamination. It is also possible to use as a menstrual collecting member a condom having passed his expiration date, because the impermeability constraints imposed on a condom used for the latter purpose are less than the one imposed for a preservative. The use of a condom has furthermore the advantage that it can be easily and discretely stored and in a hygienic manner in the bag of a female person. In such a manner a number of spare condoms can be easily transported for use during a day. A further advantage of using a condom as collecting member is that it can be easily applied to and removed from the retaining member due to the elastic properties of the condom.

The collecting member is made of expandable or elastic material because not only the member has to be applied on the body, but it must also form a reservoir having sufficient capacity to reliably store the collected menstrual flow. Also, for this purpose the condom offers a favourable solution as it has this storage property. The collecting member is preferably stored in a closed, and more preferably sealed, pocket in order to avoid contamination. Care is also preferably taken that the collecting member is clean and not contaminated when stored in the pocket. As shown in figure 3, the collecting member 3 is removably applied on the body 2. The collecting member has an entrance opening 8 and a bottom part 9. In use, the collecting member is in frictional contact with at least part of said outer 7 and inner 6 wall of the body. The collecting member is applied in such a manner to the body as to envelope the body and having its bottom part 9 crossing the entrance opening 8 of the collecting member.

In order to apply the collecting member 3 to the body, the bottom part 9 is introduced into the opening formed by the first end 4 of the body and lowered through the body so as to cross the second open end 5 of the body. During this application the collecting member can be hold by its part where the entrance opening 8 is located. When using a condom this entrance opening is generally provided with a stroke 10 formed by having locally a thicker wall dimension. This stroke facilitates the manipulation of the condom when applied on the body. Using the stroke 10 has also a hygienic advantage as the stroke does not have to be in contact with the vagina when the device is applied in the vagina.

Once the collecting member has been partially inserted in the body and having its bottom part extending out of the second end 5 of the body, the remaining part of the collecting member is fold over the first end 4 and applied against at least a part of the outer wall 7 of the body so as to envelope the body. The remaining part of the collecting member is further lowered but care is taken that the bottom part 9 remains accessible and extends through the entrance opening 8 of the collecting member. As the bottom part of the collecting member crosses the entrance opening, the bottom part remains accessible and can be used to pull on the collecting member upon removal of the device out of the vagina.

When applying the device in the vagina, the body with the collecting member is preferably somewhat folded or twisted, which is made possible by the elastic material of which the body is made. This enables to reduce temporarily the size of the device and facilitates the application into the vagina.

As the collecting member is in frictional contact with the body and as the collecting member envelops the body when applied thereon, the collecting member remains in contact with the body without the need to use any other fixing means, which provides a real comfort for the person carrying the device. As moreover the body is enveloped by the collecting member it is only the collecting member which is in direct contact with the female body, thereby considerably reducing the risk of contamination of the female body, in particular the risk for toxic shock syndrome (TSS).

Also, when removing the device from the vagina, the fact that the body is enveloped by the collecting member will cause the body to remain in contact with the collecting member when pulling on the bottom part of the collecting member. When the device has been removed from the vagina, the body can easily be removed out of the member by raising the part of the member that extends out of the body. Once the outer wall of the body has been freed from the collecting member, the latter can be completely lowered through the second end of the body. Once removed from the body, the collecting member can then be closed by applying a knot in the upper part of the collecting member thereby reducing the risk of blood spilling. Once closed the collected member can then be disposed. As the retaining body was enveloped in the collecting member it will be sufficient to clean it with some water before re-using it. Sterilisation of the retaining body is not required as the latter was and will be enveloped by the collecting member. Indeed, when re-using the device according to the invention the body will again be enveloped by a fresh collecting member and there will again be no direct contact with the female body.

Figure 4 shows a second preferred embodiment of the menstrual flow collecting device according to the invention. This embodiment distinguishes over the first embodiment in that the collecting member 3 is applied to the retaining body 2 by introducing the entrance opening 8 in the body and pushing the member inside the body so that it comes in frictional contact with the inner wall of the body. The part of the collecting member extending outside the retaining body is applied in such a manner that it is in frictional contact with at least a part of the outer wall of the body and extends over a distance larger than a length of the body so that it envelops the body.

In use, the device according to the second embodiment will be introduced into the vagina by first introducing the first end of the body. The menstrual flow can then flow through the first end to reach the bottom part of the collecting member. For removing the device, the latter will be withdrawn from the vagina by pulling on the bottom part of the collecting member.

## Claims

1. A menstrual flow collecting device comprising a retaining body and a collecting member made of expandable impermeable material, said collecting member being applied to said body, said body comprises a first and a second end located at opposite sides of said body and an outer and an inner wall, said first end being an open end, said collecting member having a bottom part, **characterised in that** said collecting member being, in use, in frictional contact with at least part of said outer and inner wall, said collecting member being applied in such a manner to said body as to envelope said body.

2. The menstrual flow collecting device as claimed in claim 1, **characterised in that** said collecting member is removably applied to said body.

3. The menstrual flow collecting device as claimed in claim 1 or 2, **characterised in that** said second end is also an open end and said collecting member has an entrance opening and being applied in such a manner to said body as to having its bottom part crossing said entrance opening.

4. The menstrual flow collecting device as claimed in anyone of the claims 1 to 3, **characterised in that** said retaining body is vessel shaped.

5. The menstrual flow collecting device as claimed in anyone of the claims 1 to 4, **characterised in that** said collecting member is disposable.

6. The menstrual flow collecting device as claimed in anyone of the claims 1 to 5, **characterised in that** said collecting member is cup shaped.

7. The menstrual flow collecting device as claimed in anyone of the claim 1 to 6, **characterised in that** said collecting member is formed by a condom.

8. The menstrual flow collecting device as claimed in claim 7, **characterised in that** said condom is one having passed his expiration date.

9. The menstrual flow collecting device as claimed in anyone of the claims 1 to 8, **characterised in that** said body is made of elastic material enabling to fold or twist the body.

10. The menstrual flow collecting device as claimed in claim 4, **characterised in that** said body is conically shaped.

11. The menstrual flow collecting device as claimed in anyone of the claims 1 to 3, **characterised in that** said body is funnel shaped.

12. The menstrual flow collecting device as claimed in anyone of the claims 1 to 3, **characterised in that** said body is tube shaped.

13. The menstrual flow collecting device as claimed in anyone of the claims 1 to 3, **characterised in that** said body is ring shaped.
